# EUROPÄISCHE PATENTANMELDUNG

(11) **EP 0 588 269 A2**
(43) Veröffentlichungstag der Anmeldung: **23.03.1994**
(21) Anmeldenummer: 93114633.6
(22) Anmeldetag: 11.09.1993
(51) Int. Cl.: A61F 13/00, A61F 15/00

(54) **Wasserdichter Wundverband**

(30) Priorität: 16.09.1992 DE 9212449 U
(71) Anmelder: Jernoiu, Justin, D-42119 Wuppertal (DE)
(72) Erfinder: Jernoiu, Justin, D-42119 Wuppertal (DE)
(74) Vertreter: Eichler, Peter, Dipl.-Ing.

(57) **Zusammenfassung**

Wasserdichte Wundverbände können aus einem über das die Wunde aufweisende Körperteil zu streifenden, wasserdichten Hüllschlauch (1) aus flexiblem Kunststoff-Plastikmaterial bestehen, der an seinem offenen Überstreifende (2) mit einem dessen Ringsumabdichtung ermöglichenden, verformbaren, wasserundurchlässigen Abdichtungsstreifen versehen ist.

Um solch einen Wundverband leicht herstellen und anlegen wie auch wiederholt verwenden zu können, dient als Abdichtungsstreifen ein am offenen Überstreifende (2) des Hüllschlauchs (1) angebrachtes elastisches Wickelband (4), dessen Länge ein Mehrfaches der Öffnungsweite des Überstreifendes (2) beträgt und das an seinem freien Ende (4') mit die fertige Bandwicklung und das davon umwickelte Hüllschlauchende (2) zusammenhaltenden Verschlußmitteln (z.B.6) versehen ist.

## Beschreibung

Die Erfindung betrifft einen wasserdichten Wundverband, der aus einem über das die Wunde aufweisende Körperteil zu streifenden wasserdichten Hüllschlauch aus flexiblem Kunststoff-Plastikmaterial besteht, der an seinem offenen Überstreifende mit einem dessen Ringsumabdichtung ermöglichenden, verformbaren, wasserundurchlässigen Abdichtungsstreifen versehen ist.

Wasserdichte Wundverbände obiger Art sind bekannt. Im Gegensatz zu den um das mit der Wunde versehene Körperteil herumzuwickelnden, mit einer Klebeinnenschicht versehenen Verbandwickeln kann bei ihnen der Kunststoffhüllschlauch über sein offenes Ende einfach über das die Wunde aufweisende Körperteil gestreift und nach entsprechendem Zusammenraffen des Überstreifendes mit Hilfe eines besonderen Klebestreifens abdichtend festgelegt werden, wobei der Klebestreifen jeweils etwa zur Hälfte auf dem Überstreifende des Plastikhüllschlauchs einerseits und auf der daneben liegenden Körperoberfläche andererseits aufgeklebt wird. Die dabei wie auch bei den vorerwähnten Verbandwickeln vorhandene Klebeschicht schließt aber eine Mehrfachverwendbarkeit solcher Wundverbände aus. Anders ist das bei dem durch das DE-GM 92 05 982 bekannten wasserdichten Wundverband, der auch aus einem über das wunde Körperteil zu streifenden wasserdichten Hüllschlauch aus Plastikmaterial besteht, jedoch an seinem offenen Überstreifende innen mit einem die Ringsumabdichtung bewirkenden, aufblasbaren Luftschlauch und außen mit einem Klettverschluß versehen ist. Ein solcher Wundverband ist wiederholt verwendbar, dafür allerdings aber auch baulich und herstellungsmäßig aufwendiger.

Der Erfindung liegt die Aufgabe zugrunde, einen wasserdichten Wundverband der eingangs erwähnten Gattung dahingehend zu verbessern und zu vervollkommnen, daß er sowohl leicht herstellbar und anlegbar ist als auch wiederholt verwendet werden kann.

Diese Aufgabe wird ausgehend von einem Wundverband der eingangs erwähnten Art erfindungsgemäß dadurch gelöst, daß als Abdichtungsstreifen ein am offenen Überstreifende des Hüllschlauchs angebrachtes elastisches Wickelband dient, dessen Länge ein Mehrfaches der Öffnungsweite des Überstreifendes beträgt und das an seinem freien Ende mit die fertige Bandwicklung und das davon umwickelte Hüllschlauchende zusammenhaltenden Verschlußmitteln versehen ist.

Ein solcher Wundverband ist von äußerst einfacher Beschaffenheit und wegen des Fehlens von abnutzungsempfindlichen Klebeschichten beliebig oft über das die regelmäßig verbundene Wunde enthaltende Körperteil zu streifen, woraufhin das Überstreifende nur noch um das betreffende Körperteil dicht herumliegend gerafft und alsdann von dem elastischen Wickelband umwickelt zu werden braucht. Da das Wickelband unverlierbar mit dem Plastik-Hüllschlauch verbunden ist, kann der Wundverband jederzeit sofort angelegt werden, ohne daß es dazu also etwa zusätzlicher Klebe- oder anderer Verschlußmittel bedarf.

Vorteilhaft besteht das Wickelband aus einem Gummiband, das am offenen Hüllschlauchende insbesondere durch Annähen befestigt ist. Das Wickelband kann aber ebenso gut auch aus elastischem Kunststoff, insbesondere einer Polyurethanfolie bestehen, die am offenen Hüllschlauchende angeschweißt ist.

Als Verschlußmittel für das Wickelband können zwei am freien Wickelbandende auf entgegengesetzten Seiten angebrachte Klettverschlußteile, nämlich der Flauschteil des Klettverschlusses einerseits und sein Hakenteil andererseits verwendet werden, wobei das Hakenteil insbesondere am äußersten Wickelbandende auf dessen Innenseite und das Flauschteil in etwa dem normalen Arm- oder Beinumfang entsprechendem Abstand davor auf der Wickelbandaußenseite angebracht sind. Hierdurch kann das Wickelband, nachdem es um das über das betreffende Körperteil gestreifte und darauf entsprechend geraffte offene Ende des Hüllschlauchs fest herumgewickelt worden ist, durch den Klettverschluß leicht zusammengehalten werden.

Als besonders vorteilhaft hat es sich erwiesen, wenn der Hüllschlauch im wesentlichen aus sogenannter medizinischer Kunststoff-Folie besteht, an der im Bereich des offenen Hüllschlauchendes eine letzteres umrandende Dichtlippe aus äußerst dünner Kunststoff-Folie angeschweißt ist. Hierdurch wird das zum Schließen des aufgestreiften Hüllschlauchendes jeweils notwendige Raffen der Kunststoff-Folie wesentlich erleichtert, weil die äußerst dünnwandige Dichtlippe sich dabei auch in ihren durch die Raffung gebildeten Überlappungsbereichen jeweils ohne störende Durchtrittskanalbildung flach aufeinanderlegt und dadurch sowohl die Umwicklung durch das Wickelband als auch die Wasserundurchlässigkeit begünstigt. Demgegenüber gewahrleistet die im übrigen die Beschaffenheit des Hüllschlauchs ausmachende medizinische Kunststoff-Folie eine solide und wirksame Schutzabdeckung der Wunde.

Für die Praxis genügt es in vielen Fällen, wenn der über das die Wunde enthaltende Körperteil zu streifende Hüllschlauch lediglich ein offenes Überstreifende mit daran befestigtem elastischen Wickelband aufweist, der Hüllschlauch also im übrigen taschen- bzw. beutelartig geschlossen ist. Ebensogut kann aber der Kunststoff-Hüllschlauch auch zwei sich im wesentlichen gegenüberliegende offene Überstreifenden besitzen, von denen ein jedes mit einem daran befestigten elastischen Wickelband und an dessen Ende vorhandenen Verschlußmitteln versehen ist. Ein solcher Wundverband kann also auch mit seinen beiden Enden über das die Wunde aufweisende Körperteil aufgestreift werden, wie das etwa bei im Oberschenkel- oder Oberarmbereich liegenden Wunden notwendig ist. Auch in diesen Fällen kann das Raffen der beiden Überstreifenden des Hüllschlauchs und deren Bandumwicklung mühelos vorgenommen werden, selbst durch eigene Einhandbedienung, wie sie etwa bei entsprechenden Oberarm-Wunden notwendig sein kann.

Nach einem weiteren Merkmal der Erfindung kann der Hüllschlauch auch zumindest bereichsweise aufblasbar und dafür mit einem geeigneten Füllventil versehen sein. Dadurch kann ein zu festes Aufliegen des Wundverbandes bzw. seines Hüllschlauchs auf dem die Wunde enthaltenden Körperteil vermieden oder aber auch eine bereichsweise Straffung des Hüllschlauchs erreicht werden, die in einigen Bedarfsfällen das Tragen des Wundverbandes noch angenehmer macht. Für dieses bereichsweise Aufblasen kann der Hüllschlauch mit einer oder mehreren aufblasbaren Schlauchrippen versehen sein, die sowohl auf der Außen- als auch Innenseite des Hüllschlauchs zusätzlich vorhanden sein können.

In der Zeichnung sind mehrere Ausführungsbeispiele erfindungsgemäß beschaffener wasserdichter Wundverbände dargestellt. Dabei zeigen
- Fig.1: einen einseitig offenen Wundverband in schaubildlicher Gesamtdarstellung,
- Fig.2: eine Seitenansicht auf das mit einem Klettverschluß versehene äußerste Ende des Hüllschlauch-Wickelbandes,
- Fig.3: die Teildraufsicht auf einen einseitig offenen Wundverband ähnlich Fig.1,
- Fig.4: einen Schnitt nach der Linie IV-IV der Fig.3,
- Fig.5: die Teilansicht eines beidendig offenen Wundverbandes,
- Fig.6 und 7: ein mit einem anderen Verschlußmittel versehenes Wickelbandende in der Seitenansicht und Draufsicht,
- Fig.8: eine weitere Ausführungsform des neuen Wundverbandes in schaubildlicher Ansicht und
- Fig.9: eine Teilschnittdarstellung eines Füllventil-Bereichs des Wundverbandes.

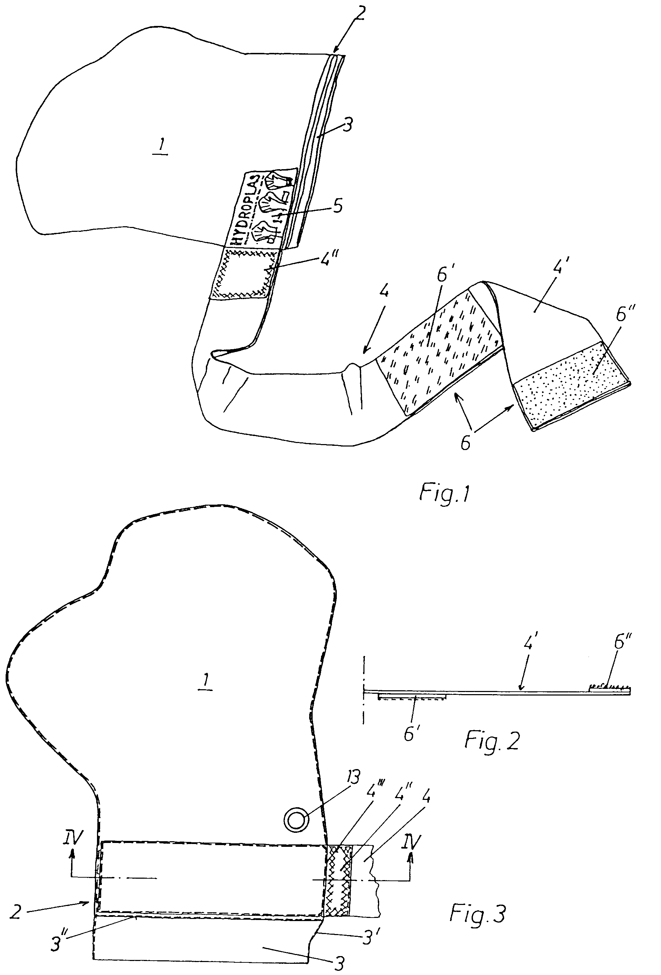

Der in Fig.1 dargestellte wasserdichte Wundverband besteht im wesentlichen aus dem über das die Wunde aufweisende Körperteil zu streifenden wasserdichten Hüllschlauch 1, der an seinem offenen Überstreifende 2 eine dessen Umrandung bildende Dichtlippe 3 sowie das hier fest angebrachte Wickelband 4 mit den an seinem freien Ende 4' angebrachten Verschlußteilen aufweist.

Der Hüllschlauch 1 besteht aus flexiblem Kunststoff-Plastikmaterial, vorzugsweise aus sogenannter medizinischer Kunststoff-Folie, die trotz ihrer Dünnwandigkeit von hinreichender Stabilität und auch gut keimfrei zu halten bzw. aufzubewahren und zu reinigen ist. Wie Fig.3 zeigt, kann der Hüllschlauch 1 aus entsprechend zugeschnittenen und miteinander verschweißten Folienteilen bestehen. Auch kann entsprechend Fig.1 auf der Hüllschlauch-Folie noch eine weitere zu Beschriftungs- bzw. Werbezwecken dienende Folie 5 aufgeschweißt sein, die auch als Einlegetasche ausgebildet sein kann.

Wie insbesondere Fig.3 zeigt, ist die das offene Überstreifende 2 des Hüllschlauchs 1 umrandende Dichtlippe 3 über die Naht 3'' an die den Hüllschlauch 1 bildende medizinische Kunststoff-Folie angeschweißt. Diese Dichtlippe 3 ist mit einem schräg nach innen weisenden Einsprung 3' versehen, der in Höhe der Befestigungsstelle 4'' des Wickelbandes 4 am Hüllschlauchende 2 liegt. Hierdurch wird sichergestellt, daß nach dem Überstreifen des Wundverbandes auf das die Wunde aufweisende Körperteil beim anschließenden Raffen des Dichtlippenrandes 3 und Anziehen des Wickelbandes 4 an der dann für etwaigen Wasserdurchtritt gefährdeten Knickstelle 4''' kein vom Inneren des Hüllschlauchs 1 nach außen durchgehender Durchtrittskanal verbleibt, weil dieser durch den Einsprung 3' des Dichtlippenrandes 3 in jedem Falle noch versperrt wird.

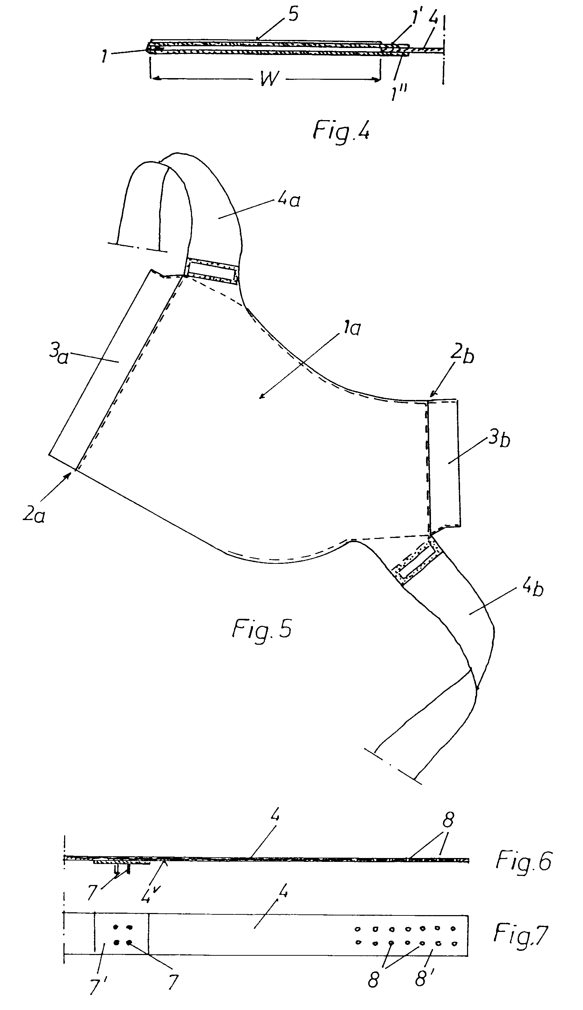

Das am offenen Überstreifende 2 des Hüllschlauchs 1 fest angebrachte Wickelband 4 besteht vorzugsweise aus einem Gummiband, das an der Stelle 4'' etwa gemäß Fig.4 mit an der Hüllschlauch-Folie 1 ausladenden Vorsprüngen 1',1'' vernäht oder ggfs. auch verschweißt ist. Die Länge dieses elastischen Wickelbandes 4 beträgt ein Mehrfaches der Öffnungsweite W des Überstreifendes 2 des Hüllschlauchs 1 (vgl. Fig.4). An seinem freien Ende 4' ist das Wickelband 4 mit es im fertig umwickelten Zustand zusammenhaltenden Verschlußmitteln 6 versehen. Es sind dies hier die beiden zu einem Klettverschluß gehörenden Teile, nämlich dessen Flauschteil 6' einerseits und dessen Hakenteil 6'' andererseits. Während das Hakenteil 6'' entsprechend Fig.1 und 2 am äußersten Wickelbandende auf dessen Innenseite 4^{IV} angebracht ist, sitzt das Flauschteil 6' auf der Außenseite 4^{V} des Wickelbandes, und zwar in einem etwa dem normalen Arm- oder Beinumfang entsprechenden Abstand vom Hakenteil 6''.

Zum Anlegen des vorbeschriebenen Wundverbandes braucht dieser mit seinem offenen Ende 2 soweit über das die Wunde aufweisende Körperteil gestreift zu werden, daß dabei die Wunde von dem Hüllschlauch 1 allseitig umgeben wird. Sodann wird das offene Hüllschlauchende 2, insbesondere seine Dichtlippe 3, unter entsprechendem Raffen fest auf die betreffende Körperoberfläche gedrückt und sodann das Wickelband 4 entsprechend fest herumgewickelt, bis schließlich dessen Ende 4' mit dem darauf innenseitig sitzenden Klettverschluß-Hakenteil 6'' auf den oberseitig zugänglich gebliebenen Flauschteil 6' des Klettverschlusses gedrückt und dadurch die fertige Bandwicklung sowie das davon umwickelte Hüllschlauchende 2 wasserabdichtend zusammengehalten werden.

Der in Fig.5 dargestellte, insbesondere für Oberarm- bzw. Oberschenkel-Wunden bestimmte Wundverband besitzt auch einen aus Kunststoff-Plastikmaterial bestehenden Hüllschlauch 1, der jedoch zwei sich im wesentlichen gegenüberliegende offene Überstreifenden 2a,2b mit je einem daran befestigten elastischen Wickelband 4a,4b sowie weiterhin auch je eine aus äusserst dünner Kunststoff-Folie bestehende, die Überstreifenden umrandende Dichtlippe 3a bzw. 3b aufweist. Die Wickelbänder 4a,4b sind an ihren äußersten Enden mit entsprechenden Verschlußmitteln versehen, beispielsweise also auch mit den vorbeschriebenen Klettverschlußteilen, um die nach Anlegen des Wundverbandes fertigen Bandwicklungen und die davon umwickelten Hüllschlauchenden 2a bzw. 2b zusammenhalten zu können.

Die Verschlußteile müssen nicht unbedingt als Klettverschlüsse ausgebildet sein. Vielmehr zeigen die Fig.6 und 7 eine Ausführungsform für Wickelband-Endverschlüsse, bei denen die Verschlußmittel aus je einem am freien Wickelbandende angebrachten, mit Einstecknadeln 7 bestückten Nadelbereich 7' und einem damit zusammenwirkenden entsprechend angeordnete Einstecklöcher 8 aufweisenden Einstecklochbereich 8' bestehen. Letzterer ist am äußersten Wickelbandende vorgesehen, während der Nadelbereich 7' in entsprechendem Abstand davon auf der Außenseite 4^{V} des Wickelbandes 4 angeordnet ist. Es versteht sich, daß in diesem Falle je nach Arm- oder Beinumfang das fertig um das offene Überstreifende des Hüllschlauchs 1 gewickelte Band 4 mit den an seinem äußersten Ende gelegenen, entsprechend passenden Einstecklöchern 8' auf die außen- bzw. bandoberseitig vorspringenden Einstecknadeln 7 aufgesteckt und dadurch die Bandwicklung gesichert bzw. zusammengehalten werden kann.

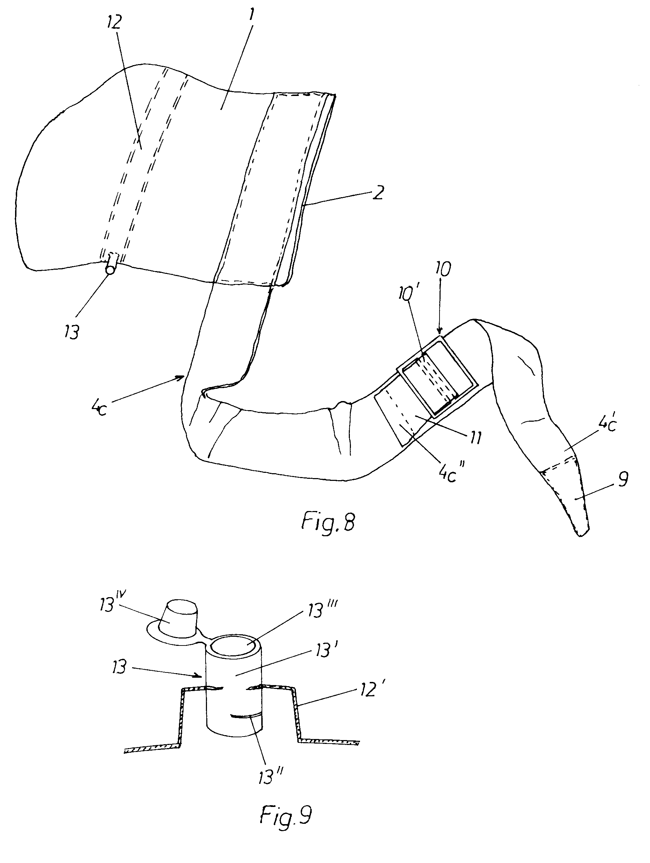

Bei dem in Fig.8 dargestellten Wundverband besteht das Wickelband 4c aus einem am Hüllschlauch 1 angeschweißten elastischen Kunststoffband, vorzugsweise einer Polyurethanfolie. Auch die am freien Wickelbandende 4c' vorhandenen Verschlußmittel sind hier von anderer Beschaffenheit, nämlich als Gürtel-Schnallen-Verschluß ausgebildet, wobei das äußerste Wickelbandende als Gürteleinsteckzunge 9 ausgebildet und die zugehörige Schnalle 10 in entsprechendem Abstand davor auf der Außenseite des Wickelbandes 4c vorgesehen ist. Die Befestigung der Schnalle 10 kann hier über die um deren Mittelsteg 10' herumgeschlagene und auf dem Wickelband 4c an der Stelle 4c'' verschweißte Kunststofflasche 11 erfolgen.

Eine weitere Besonderheit bei der in Fig.8 dargestellten Ausführungsform besteht darin, daß hier der Hüllschlauch 1 mit einer aufgeschweißten Schlauchrippe 12 versehen ist, die über das Füllventil 13 mit Luft aufblasbar ist. Durch eine solche aufgeblasene oder auch mehrere solcher Schlauchrippen 12 kann eine entsprechende Aufblähung des Hüllschlauchs 1 erzielt werden, die seine Anpassungsfähigkeit und Tragannehmlichkeit noch verbessern hilft. Dabei kann das Füllventil 13 beispielsweise von der in Fig.9 dargestellten Beschaffenheit sein, nämlich aus einem in die Schlauchrippenwand 12' eingeschweißten Kunststoffröhrchen 13' bestehen, das unterseitig geschlossen ist und einen darüber gelegenen, selbsttätig sich öffnenden und schließenden Sperrschlitz 13'' aufweist. Die obere Füllöffnung 13''' des Füllventils kann nach seiner jeweiligen Betätigung durch den Füllstopfen 13^{IV} luftdicht verschlossen werden. Anstelle einer oder mehrerer solcher aufblasbarer Schlauchrippen 12 kann der Schlauch 1 aber auch insgesamt aufblasbar und dazu mit einem Füllventil 13 versehen sein, wie das bei dem in Fig. 3 dargestellten Wundverband durch den darin eingezeichneten Doppelkreis 13 angedeutet ist.

Das am offenen Überstreifende 2 des Hüllschlauchs 1 angebrachte Wickelband 4 bzw. 4a,4b oder 4c muß damit nicht unbedingt unlösbar fest verbunden sein. Vielmehr kann hierfür auch eine lösbare Verbindung ausreichen, wo also das Wickelband 4 am Hüllschlauchende 2 etwa auch über einen Klettverschluß, Knopfloch- oder Gürtel-Schnallen-Verschluß verbunden wäre. Wohl aber ist für die vorliegende Erfindung wesentlich, daß das elastische Wickelband am Hüllschlauch 1 unverlierbar angebracht ist und dadurch der wasserdichte Wundverband unmittelbar nach seinem Anlegen wasserdicht verschlossen werden kann, ohne daß es dazu besonderer Abdichtungs- oder Klebestreifen auf der Innenseite der Hüllschlauchenden bedarf. Insbesondere wird in Verbindung mit den äußerst dünnwandig beschaffenen, die Hüllschlauchenden umrandenden Dichtlippen 3 bzw. 3a,3b auch sichergestellt, daß an diesen Stellen die Kunststoff-Folie eng bzw. dicht gerafft werden kann, so daß bei deren anschließendem Umwickeln mittels der elastischen Wickelbänder 4 bzw. 4a,4b hier ein wasserdichter Abschluß erzielt werden kann. Da keinerlei abnutzungsgefährdete Klebestellen vorhanden sind, kann der neue Wundverband allfällig auch leicht gereinigt und beliebig oft verwendet werden. Im übrigen ist der neuartige Wundverband nicht etwa nur zur wasserdichten Abdeckung menschlicher Körperwundstellen geeignet, sondern ebensogut und vorteilhaft auch für an Tieren, z.B. Pferden oder auch Hunden vorhandene Wund- bzw. Verbandstellen verwendbar.

## Patentansprüche

1. Wasserdichter Wundverband, bestehend aus einem über das die Wunde aufweisende Körperteil zu streifenden, wasserdichten Hüllschlauch (1) aus flexiblem Kunststoff-Plastikmaterial, der an seinem offenen Überstreifende (2) mit einem dessen Ringsumabdichtung ermöglichenden, verformbaren, wasserundurchlässigen Abdichtungsstreifen versehen ist, **dadurch gekennzeichnet**, daß als Abdichtungsstreifen ein am offenen Überstreifende (2) des Hüllschlauchs (1) angebrachtes elastisches Wickelband (4) dient, dessen Länge ein Mehrfaches der Öffnungsweite (W) des Überstreifendes (2) beträgt und das an seinem freien Ende (4') mit die fertige Bandwicklung und das davon umwickelte Hüllschlauchende (2) zusammenhaltenden Verschlußmitteln (z.B.6) versehen ist.

2. Wundverband nach Anspruch 1, **dadurch gekennzeichnet**, daß das Wickelband aus einem Gummiband (4) besteht, das am offenen Hüllschlauchende (2) insbesondere angenäht ist.

3. Wundverband nach Anspruch 1, **dadurch gekennzeichnet**, daß das Wickelband (4) aus elastischem Kunststoff, insbesondere einer Polyurethanfolie besteht, die am offenen Hüllschlauchende (2) angeschweißt ist.

4. Wundverband nach Anspruch 2 oder 3, **dadurch gekennzeichnet**, daß die Verschlußmittel (z.B.6) aus zwei am freien Wickelbandende (4') auf entgegengesetzten Seiten angebrachten Klettverschlußteilen, nämlich dem Flauschteil (6') einerseits und dem Hakenteil (6'') andererseits bestehen, wobei insbesondere das Hakenteil (6'') am äußersten Wickelbandende auf dessen Innenseite (4^{IV}) und das Flauschteil (6') in etwa dem normalen Arm- oder Beinumfang entsprechendem Abstand davor auf der Wickelbandaussenseite (4^{V}) angebracht sind.

5. Wundverband nach einem der Ansprüche 1 bis 4, **dadurch gekennzeichnet**, daß der Hüllschlauch (1) im wesentlichen aus sogenannter medizinischer Kunststoff-Folie besteht, an die im Bereich des offenen Hüllschlauchendes (2) eine letzteres umrandende Dichtlippe (3) aus äußerst dünner Kunststoff-Folie angeschweißt ist.

6. Wundverband nach Anspruch 5, **dadurch gekennzeichnet**, daß der Dichtlippenrand (3) in Höhe der Befestigungsstelle (4'') des Wickelbandes (4) am offenen Hüllschlauchende (2) schräg nach innen einspringt.

7. Wundverband nach einem der Ansprüche 1 bis 6, **dadurch gekennzeichnet**, daß der Hüllschlauch (1) zwei sich im wesentlichen gegenüberliegende offene Überstreifenden (2a, 2b) mit je einem daran befestigten elastischen Wickelband (4a bzw. 4b) aufweist (Fig.5).

8. Wundverband nach einem der Ansprüche 1 bis 7, **dadurch gekennzeichnet**, daß die Verschlußmittel aus je einem am freien Wickelbandende (4') angebrachten Nadel- und damit zusammenwirkenden Einsteckloch-Bereich (7' bzw. 8') bestehen, von denen letzterer am äußersten Wickelbandende und der Nadelbereich (7') in entsprechendem Abstand davor auf der Außenseite (4^{V}) des Wickelbandes (4) vorgesehen sind (Fig.6,7).

9. Wundverband nach einem der Ansprüche 1 bis 7, **dadurch gekennzeichnet**, daß die Verschlußmittel aus einem am freien Wickelbandende (4c') angebrachten Gürtel-Schnallen-Verschluß (9,10) bestehen, wobei das äußerste Wickelbandende (4c') als Gürteleinsteckzunge (9) ausgebildet und die zugehörige Schnalle (10) in entsprechendem Abstand davor auf der Außenseite des Wickelbandes (4c) vorgesehen ist (Fig.8).

10. Wundverband nach einem der Ansprüche 1 bis 9, **dadurch gekennzeichnet**, daß der Hüllschlauch (1) zumindest bereichsweise aufblasbar und dafür mit einem geeigneten Füllventil (13) versehen ist (Fig.3,8 und 9).

11. Wundverband nach Anspruch 10, **dadurch gekennzeichnet**, daß der Hüllschlauch (1) mit einer oder mehreren aufblasbaren Schlauchrippen (12) versehen ist (Fig.8).
